(19) Europäisches Patentamt
European Patent Office
Office européen des brevets

(11) Veröffentlichungsnummer: **0 126 933**
**A1**

(12) # EUROPÄISCHE PATENTANMELDUNG

(21) Anmeldenummer: **84104193.2**

(22) Anmeldetag: **13.04.84**

(51) Int. Cl.³: **C 07 D 487/04**
A 61 K 31/41

(30) Priorität: **23.04.83 DE 3314843**

(43) Veröffentlichungstag der Anmeldung: **05.12.84 Patentblatt 84/49**

(84) Benannte Vertragsstaaten: **AT BE CH DE FR GB IT LI NL SE**

(71) Anmelder: **Beiersdorf Aktiengesellschaft**
**Unnastrasse 48**
**D-2000 Hamburg 20(DE)**

(72) Erfinder: **Cohnen, Erich, Dr.**
**Moorende 74**
**D-2155 Jork(DE)**

(72) Erfinder: **Armah, Ben, Dr.**
**Milcher Strasse 9d**
**D-2000 Hamburg 52(DE)**

(54) **Tetrahydro-1H-pyrazolo(5,1-a)isoindole, Verfahren zu deren Herstellung, diese Verbindungen enthaltende Zubereitungen und Zwischenprodukte.**

(57) Neue substituierte Tetrahydro-1H-pyrazolo[5,1-a] isoindole der Formel I

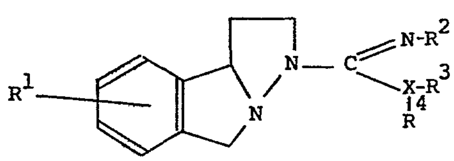

(I)

in der

$R^1$ ein Wasserstoff- oder Halogenatom oder eine Alkoxygruppe mit 1 bis 4 Kohlenstoffatomen,

$R^2$, $R^3$, $R^4$, die gleich oder verschieden sein können, ein Wasserstoffatom oder eine Alkylgruppe mit 1 bis 4 Kohlenstoffatomen oder

$R^2$ und $R^3$ zusammen die Äthylengruppe und

X ein Stickstoffatom oder eine Methingruppe bedeuten sowie deren Säureadditionssalze besitzen eine blutdrucksteigernde Wirkung und können als Antihypotonica verwendet werden.

EP 0 126 933 A1

BEIERSDORF AKTIENGESELLSCHAFT

HAMBURG

Tetrahydro-1H-pyrazolo[5,1-a]isoindole, Verfahren zu deren Herstellung, diese Verbindungen enthaltende Zubereitungen und Zwischenprodukte.

Gegenstand der Erfindung sind neue substituierte Tetrahydro-1H-pyrazolo[5,1-a]isoindole der Formel I

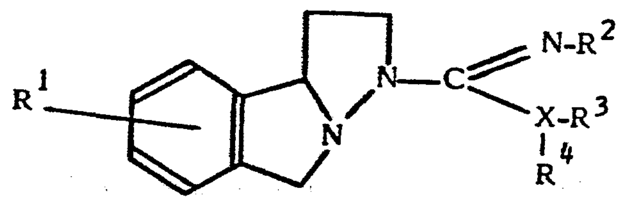

(I)

in der

$R^1$ ein Wasserstoff- oder Halogenatom oder eine Alkoxygruppe mit 1 bis 4 Kohlenstoffatomen,

$R^2$, $R^3$, $R^4$, die gleich oder verschieden sein können, ein Wasserstoffatom oder eine Alkylgruppe mit 1 bis 4 Kohlenstoffatomen oder

$R^2$ und $R^3$ zusammen die Äthylengruppe und

X ein Stickstoffatom oder eine Methingruppe bedeuten

und ihre Säureadditionssalze, Verfahren zu ihrer Herstellung und ihre Verwendung in pharmazeutischen Präparaten.

Ferner betrifft die Erfindung neue Verbindungen der Formel II

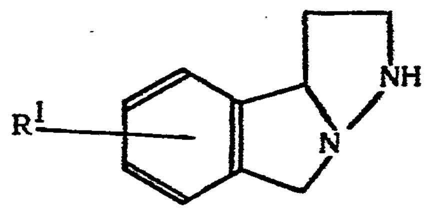

(II)

in der $R^1$ die oben angegebene Bedeutung hat und ihre Salze, Verfahren zu ihrer Herstellung sowie ihre Verwendung als Zwischenprodukte für die Herstellung der erfindungsgemäßen Verbindungen gemäß Anspruch 1.

Obgleich pharmazeutisch verträgliche Salze der neuen Verbindungen der Formel I bevorzugt sind, liegen alle Säureadditionssalze innerhalb des Bereichs der Erfindung. Alle Säureadditionssalze sind wertvoll zur Herstellung der Basen, selbst wenn das spezielle Salz nur als Zwischenprodukt gewünscht wird, wie z.B., wenn das Salz nur für Zwecke der Reinigung oder Identifizierung gebildet wird, oder wenn es als ein Zwischenprodukt bei der Herstellung eines pharmazeutisch verträglichen Salzes, wie z.B. durch Ionenaustauschverfahrensweisen, verwendet wird.

Die neuen Verbindungen der allgemeinen Formeln I und II enthalten asymmetrische Kohlenstoffatome. Daher sind auch die verschiedenen optischen Isomeren sowie die Diastereoisomere Gegenstand der Erfindung ebenso wie die Additionssalze dieser Verbindung mit Säuren. Racemate können nach an sich bekannten Methoden in ihre optischen Antipoden aufgetrennt werden.

Die erfindungsgemäßen Alkylgruppen sowie die Alkylteile der Alkoxygruppen können geradkettig oder verzweigt sein und sind vorzugsweise Methyl- oder Ethylgruppen.

Bevorzugte Alkoxygruppen sind die Methoxy- und Ethoxygruppe.

Halogen ist vorzugsweise Fluor, Chlor oder Brom.

Besonders bevorzugte Alkygruppen sind die Methyl- und Ethylgruppe.

$R^1$ ist vorzugsweise Wasserstoff. Substituenten $R^1$, die nicht Wasserstoff bedeuten, befinden sich bevorzugt in der 4-, 6- oder 7-Stellung des Phenylrestes.

X ist vorzugsweise Stickstoff.

Die erfindungsgemäßen Carboximidamide der Formel I, in der X Stickstoff und $R^2$, $R^3$ und $R^4$ Wasserstoff oder Alkyl bedeuten und $R^1$ die angegebene Bedeutung hat, werden besonders bevorzugt.

Auch die Imidazoline der Formel I, in der X Stickstoff ist und $R^2$ und $R^3$ zusammen die Äthylengruppe bedeuten und $R^1$ und $R^4$ die angegebene Bedeutung haben, werden bevorzugt.

Ferner werden Acetamido-Verbindungen der Formel I bevorzugt, in der X die Methingruppe bedeutet, $R^2$, $R^3$ und $R^4$ Wasserstoff oder Alkyl sind und $R^1$ die angegebene Bedeutung hat.

Die Verbindung 2,3,3a,8-Tetrahydro-1H-pyrazolo[5,1-a]isoindol-1-carboximidamid zeichnet sich durch einen hohen therapeutischen Effekt aus und wird besonders bevorzugt.

Die erfindungsgemäßen neuen Verbindungen und ihre Säureadditionssalze besitzen wertvolle therapeutische Eigenschaften. Sie zeichnen sich insbesondere durch eine lang anhaltende blutdrucksteigernde Wirkung aus und können daher als Antihypotonica zur Behandlung der Hypotonie und orthostatischen Dysregulation verwendet werden.

Die neuen Verbindungen steigern bei einer Dosierung von 0,3 - 3.0 mg/kg i.v. den Blutdruck von narkotisierten Katzen um 10 - 30 %; bei normotonen Ratten kommt es nach 1 - 10 mg/kg s.c. zu einer 30%igen Blutdrucksteigerung, die über 6 Stunden anhält.

Die Verbindungen können oral oder parenteral angewendet werden. Die Dosierung für die orale Anwendung liegt beim Menschen bei 0,1 bis 50 mg, vorzugsweise 1 bis 10 mg, pro Tag. Zweckmäßigerweise werden die Substanzen in unterteilten Dosen, beispielsweise zweimal täglich, verabreicht.

Weiterhin können die erfindungsgemäßen Substanzen infolge ihrer α-sympathicomimetischen, vasokonstriktorischen Wirkung als Rhinologica, insbesondere in Form von Nasentropfen, verwendet werden. Die Dosierungen betragen bei der bevorzugten Konzentration von 1 - 10 mg/ml, entsprechend einer 0,1 - 1,0%igen wäßrigen Lösung, vorzugsweise mehrere Tropfen pro Nasenloch mehrmals täglich.

Gemäß der Erfindung werden pharmazeutische Zusammensetzungen geschaffen, die die Verbindung gemäß Anspruch 1 oder deren pharmazeutisch verträgliche Salze zusammen mit einem pharmazeutisch verträglichen Verdünnungsmittel oder Träger enthalten.

Die Verbindungen gemäß der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können oral in Form von Tabletten, Dragees, Sirups, Suspensionen und Flüssigkeiten, oder parenteral in Form von Lösungen oder Suspensionen verabreicht werden. Oral zu verabreichende

Präparate können ein oder mehrere Zusätze, wie Süßungsmittel, Aromatisierungsmittel, Farbstoffe und Konservierungsmittel, enthalten. Tabletten können den Wirkstoff mit üblichen pharmazeutisch verträglichen Hilfsmitteln vermischt enthalten, zum Beispiel inerten Verdünnungsmitteln wie Calciumcarbonat, Natriumcarbonat, Lactose und Talk, Granulierungsmitteln und Mitteln, die den Zerfall der Tabletten bei oraler Verabreichung fördern, wie Stärke oder Alginsäure, Bindemitteln wie Stärke oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Ethanol, Propylenglycol, Ether des Tetrahydrofurylalkohols und Wasser.

Die Tabletten können nach bekannten Arbeitsweisen überzogen werden, um den Zerfall und die Resorption im Magen-Darmtrakt zu verzögern, wodurch die Aktivität des Wirkstoffs sich über eine längere Zeitspanne erstrecken kann. Ebenso kann in den Suspensionen der Wirkstoff mit Hilfsmitteln vermischt sein, die für die Herstellung solcher Zusammensetzungen üblich sind, zum Beispiel Suspendiermitteln wie Methylcellulose, Tragacanth oder Natriumalginat, Netzmitteln wie Lecithin, Polyethylenstearat und Polyoxyethylensorbitanmonooleat, und Konservierungsmitteln wie Ethylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert. Die pharmazeutischen Präparate können den Wirkstoff in einer Menge von 0,1 bis 90 %, insbesondere 1 bis 90 %, enthalten, wobei der Rest ein Trägerstoff oder Zusatzstoff ist. Im Hinblick auf die Herstellung und Verabreichung werden feste Präparate, wie Tabletten und Kapseln, bevorzugt. Vorzugsweise enthalten die Präparate den Wirkstoff in einer Menge von 5 mg.

Ein Verfahren zur Herstellung der Verbindungen der Formel I ist dadurch gekennzeichnet, daß man Verbindungen der Formel II

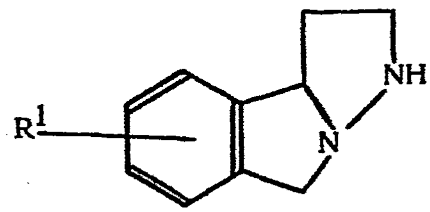

(II)

in der $R^1$ die oben angegebene Bedeutung hat, mit Verbindungen der allgemeinen Formel (III) umsetzt,

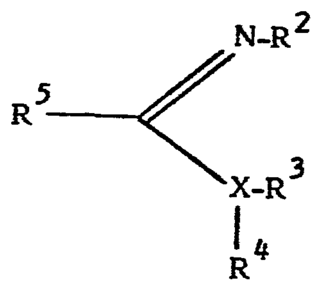

(III)

in der $R^2$, $R^3$ und $R^4$ die oben angegebene Bedeutung haben und in der $R^5$ eine nucleophil austauschbare Gruppe wie die Alkylthio- oder Alkoxygruppe bedeutet.

Bevorzugte Gruppen $R^5$ sind die Methylthio- und Methoxygruppe. Die Reaktion wird mit einem Hydrohalogenid von (III) in einem Alkohol, vorzugsweise Amylalkohol als Lösungsmittel bei Siedetemperatur durchgeführt.

Die Ausgangsverbindungen der Formel III sind bekannt oder nach bekannten Verfahren erhältlich.

Ein weiteres Verfahren zur Herstellung der erfindungsgemäßen Verbindungen der Formel I, in der $R^2$ Wasserstoff und X Stickstoff bedeutet, ist dadurch gekennzeichnet, daß man Verbindungen der Formel II, mit der angegebenen Bedeutung von $R^1$, mit Cyanamid oder mit substituierten Cyanamiden der Formel $NCNR^3R^4$, in der $R^3$ und $R^4$ die oben angegebene Bedeutung besitzen, umsetzt. Die Reaktion wird vorzugsweise in Alkoholen mit Säureadditionssalzen der Verbindungen der Formel II bei Siedetemperatur durchgeführt. Besonders geeignet ist n-Amylalkohol.

Die als Ausgangsverbindungen verwendeten substituierten Cyanamide sind bekannt oder nach bekannten Verfahren erhältlich.

Das Verfahren zur Herstellung der als Zwischenprodukte verwendeten Verbindungen der allgemeinen Formel (II) ist dadurch gekennzeichnet, daß man 2-Brommethylzimtsäure-alkylester der allgemeinen Formel (IV)

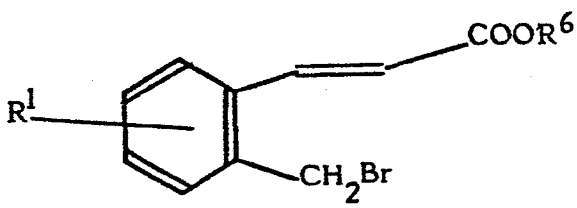

(IV)

in der $R^1$ die oben angegebene Bedeutung hat und $R^6$ eine Methyl- oder Ethylgruppe bedeutet, mit t-Butylcarbazat zu substituierten Isoindolinen der Formel (V)

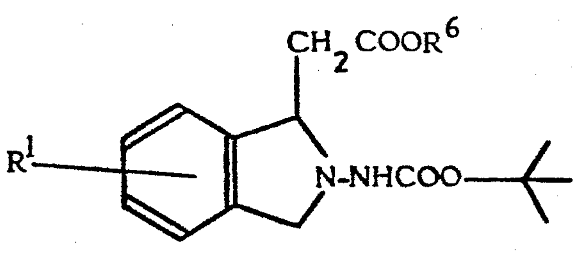

(V)

umsetzt und diese anschließend mit Säuren zu 2-Oxo-2,3,3a,8-tetrahydro-1H-pyrazolo/5,1-a/isoindolen der Formel (VI)

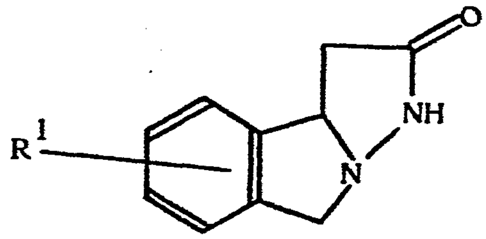

(VI)

cyclisiert. Die Lactame (VI) werden durch Reduktion in die Verbindungen der Formel (II) überführt.

Die Reaktion des ungesättigten Bromids (IV) mit t-Butylcarbazat findet bei Raumtemperatur in geeigneten Lösungsmitteln wie beispielsweise Dimethylformamid, Dimethylacetamid und N-Methylpyrrolidon statt. Geeignete Säuren sind Mineralsäure, insbesondere Salzsäure. Als Reduktionsmittel für die Reaktion (VI) → (II) sind Metallhydride, beispielsweise Lithiumaluminiumhydrid, besonders geeignet.

Die Ausgangsverbindungen der Formel IV sind bekannt oder können nach bekannten Verfahren erhalten werden.

Die Verbindungen der allgemeinen Formeln I und II können entweder als Basen oder in Form ihrer Salze aus den Reaktionsgemischen isoliert werden. Sie lassen sich als Basen mit geeigneten anorganischen oder organischen Säuren nach bekannten Verfahren in die Salze überführen.

Bevorzugt werden physiologisch verträgliche Salze der Verbindung der Formel I. Hierfür sind als anorganische Säuren beispielsweise Halogenwasserstoffsäuren, zum Beispiel Salzsäure oder Schwefelsäure und als organische Säuren zum Beispiel Fumarsäure, Maleinsäure, Zitronensäure und Weinsäure geeignet. Zur Herstellung wird die heiße alkalische Lösung der Base mit der alkoholischen Lösung einer geeigneten Säure versetzt und man erhält nach Etherzusatz das Salz.

Diastereoisomere können in bekannter Weise aufgrund der physikalisch-chemischen Unterschiede ihrer Bestandteile in ihre racemischen Modifikationen getrennt werden. Racemate können nach bekannten Methoden getrennt werden, beispielsweise durch Umkristallisieren in optisch aktiven Lösungsmitteln, durch Mikroorganismen oder Reaktion mit einer optisch aktiven Säure, die mit der racemischen Verbindung ein Salz bildet, Trennung der Diastereoisomere durch fraktionierte Kristallisation und Freisetzung der Enantiomeren durch geeignete Mittel. Besonders geeignete optisch aktive Säuren sind beispielsweise die d- und l-Formen der Weinsäure, Diorthotoloylweinsäure, Äpfelsäure, Mandelsäure, Kamphersulfonsäure oder Chininsäure. Vorteilhafterweise wird der aktivere der Antipoden isoliert. Gemäß der Erfindung ist es jedoch auch möglich, die reinen Enantiomeren durch asymmetrische Synthese zu erhalten.

Die folgenden Beispiele dienen zur Erläuterung der Erfindung:

## Beispiel 1

2,3,3a,8-Tetrahydro-1H-pyrazolo/5,1-a/isoindol-1-carboximidamid

3,92 g (0,02 mol) 2,3,3a8-Tetrahydro-1H-pyrazolo/5,1-a/isoindol-hydrochlorid und 0,95 g Cyanamid werden in 20 ml n-Amylalkohol 2 Stunden zum Sieden erhitzt. Nach Abdestillieren des Lösungsmittels nimmt man den Rückstand in siedendem Ethanol auf, setzt Aktivkohle zu und dampft nach Filtration im Vakuum ein. Nach Umkristallisation aus Isopropanol/Essigester erhält man 2,4 g des Hydrochlorids des 2,3,3a,8-Tetrahydro-1H-pyrazolo/5,1-a/isoindol-1-carboximidamid. Fp. 233 - 234°C.

Analog Beispiel 1 wurden folgende Verbindungen der Formel I hergestellt:

| Beispiel | $R^1$ | $R^2$ | $R^3$ | $R^4$ | X | Fp.°C | Salz |
|---|---|---|---|---|---|---|---|
| 2 | H | H | $CH_3$ | $CH_3$ | N | 181-83 | Fumarat |
| 3 | 4-Cl | H | H | H | N | 238-40 | HCl |
| 4 | 7-Cl | H | H | H | N | 270-72 | HCl |
| 5 | 6-$OCH_3$ | H | H | H | N | 280-82 | HCl |

Beispiel 6

1-(2-Imidazolin-2-yl)-2,3,3a,8-tetrahydro-1H-pyrazolo/5,1-a/isoindol

4,2 g (0.026 mol) 2,3,3a,8-Tetrahydro-1H-pyrazolo/5,1-a/isoindol und 6,3 g (0.026 mol)2-Methylthio-imidazolin-hydrojodid werden in 50 ml n-Amylalkohol 1,5 Stunden zum Sieden erhitzt. Nach Abdampfen des Lösungsmittels wird der Rückstand in Methanol aufgenommen, mit Aktivkohle behandelt und schließlich aus Aceton kristallisiert. Man erhält 2,8 g des Hydrojodids von 1-(2-Imidazolin-2-yl)-2,3,3a,8-tetrahydro-1H-pyrazolo/5,1-a/isoindol. Der Schmelzpunkt beträgt nach nochmaliger Umkristallisation aus Isopropanol 238-240°C (Z.).

Analog Beispiel 6 wurden folgende Carboximidamide der Formel I mit der S-Methyl-isothiuroniumsalzen der Formel III hergestellt:

N-Methyl-(2,3,3a,8-tetrahydro-1H-pyrazolo/5,1-a/isoindol-1)-carboximidamid, ($R^2$ =Methyl)

$N^1$,$N^2$-Dimethyl-(2,3,3a,8-tetrahydro-1H-pyrazolo/5,1-a/isoindol-1)-carboximidamid ($R^2$, $R^3$ = Methyl)

Beispiel 7

1-Acetimido-2,3,3a,8-tetrahydro-1H-pyrazolo/5,1-a/isoindol-hydrochlorid

3,1 g (0,019 mol) 2,3,3a,8-Tetrahydro-1H-pyrazolo/5,1-a/isoindol und 3,3 g (0,03 mol) Acetimidoethylether-hydrochlorid werden in 100 ml absolutem Ethanol 15 Stunden bei Raumtemperatur gerührt. Nach Entfernen des Ethanols wird der Rückstand von 1-Acetimido-2,3,3a,8-tetrahydro-1H-pyrazolo/5,1-a/isoindol-hydrochlorid mit Essigester/Isopropanol kristallisiert. Fp. 255 - 257°C (Z).

Beispiel 8

2,3,3a,8-Tetrahydro-1H-pyrazolo/5,1-a/isoindol-hydrochlorid

7,0 g (0,04 mol) 2-Oxo-2,3,3a,8-tetrahydro-1H-pyrazolo/5,1-a/isoindol und 3,8 g Lithiumaluminiumhydrid in 150 ml absolutem Tetrahydrofuran werden 4 Stunden zum Sieden erhitzt. Nach Abkühlen wird das überschüssige Hydrid mit 30%iger NaOH zerstört. Man filtriert den Niederschlag ab, dampft das Tetrahydrofuran ab und nimmt den Rückstand in äthanolischer Salzsäure auf. Nach Umkristallisation aus Isopropanol erhält man 4,0 g 2,3,3a,8-Tetrahydro-1H-pyrazolo/5,1-a/isoindol-hydrochlorid. Fp. 166-167°C (Z).

Analog Beispiel 8 wurden folgende Verbindungen der Formel II hergestellt:

4-Chlor-2,3,3a,8-tetrahydro-1H-pyrazolo/5,1-a/-isoindol-hydrochlorid

7-Chlor-2,3,3a,8-tetrahydro-1H-pyrazolo/5,1-a/-isoindol-hydrochlorid

6-Methoxy-2,3,3a,8-tetrahydro-1H-pyrazolo/5,1-a/-isoindol-hydrochlorid

## Beispiel 9

2-Oxo-2,3,3a,8-tetrahydro-1H-pyrazolo/5,1-a/isoindol-hydrochlorid

20,0 g (0,062 mol) N-(t-Butyloxycarbonylamino)-2,3-dihydro-1H-isoindol-1-essigsäureäthylester in 50 ml Chloroform werden unter kräftigem Rühren 15 Stunden mit 200 ml konzentrierter Salzsäure behandelt. Man entfernt die Lösungsmittel im Vakuum und kristallisiert aus Isopropanol/Essigester um. Ausbeute: 9 g 2-Oxo-2,3,3a,8-tetrahydro-1H-pyrazolo/5,1-a/isoindol-hydrochlorid. Fp. 216-218°C (Z).

Analog Beispiel 9 wurden folgende Verbindungen der Formel VI hergestellt:

2-Oxo-4-Chlor-2,3,3a,8-tetrahydro-1H-pyrazolo-/5,1-a/isoindol-hydrochlorid
Fp. 242-244°C (Z.)

2-Oxo-7-chlor-2,3,3a,8,tetrahydro-1H-pyrazolo/5,1-a/isoindol-hydrochlorid
Fp. 222-224°C (Z.)

2-Oxo-6-methoxy-2,3,3a,8-tetrahydro-1H-pyrazolo-/5,1-a/isoindol-hydrochlorid
Fp. 218-220°C (Z.)

## Beispiel 10

N-(t-Butyloxycarbonylamino)-2,3-dihydro-1H-isoindol-1-essigsäureäthylester

21,8 g (0,08 mol) 2-Bromomethyl-zimtsäure-äthylester und 26,4 g (0,20 mol) t-Butylcarbazat werden in 300 ml Dimethylformamid mehrere Tage bis zur vollständigen Umsetzung bei Raumtemperatur gerührt. Man dampft das Dimethylformamid in Vakuum ab, verteilt den Rückstand zwischen Wasser und Chloroform und reinigt den Rückstand der organischen Phase durch präparative HPLC an Kieselgel. Ausbeute 22,0 g N-(t-Butyloxycarbonylamino)-2,3-dihydro-1H-isoindol-1-essigsäureäthylester (Öl).

Analog Beispiel 10 wurden folgende Verbindungen der Formel V hergestellt:

N-(t-Butoxycarbonylamino)-4-chlor-2,3-dihydro-1H-isoindol-1-essigsäureäthylester

N-(t-Butoxycarbonylamino)-7-chlor-2,3-dihydro-1H-isoindol-1-essigsäureäthylester

N-(t-Butoxycarbonylamino)-6-methoxy-2,3-dihydro-1H-isoindol-1-essigsäureäthylester

Beispiel 11

Herstellung von Tabletten

Tabletten, die die im folgenden genannten Bestandteile enthalten, lassen sich in bekannter Weise herstellen. Sie können zur Behandlung hypotoner Zustände in einer Dosis von 1 bis 2 Tabletten einmal täglich verwendet werden.

| | |
|---|---|
| 2,3,3a,8-Tetrahydro-1H-pyrazolo/5,1-a/iso-indol-1-carboximidamid | 5 mg |
| Lactose | 75 mg |
| Maisstärke | 10 mg |
| Mikrokristalline Cellulose | 8 mg |
| Polyvinylpyrrolidon | 1 mg |
| Magnesiumstearat | 0,5 mg |
| hochdisperses Siliziumdioxid | 0,5 mg |

## Beispiel 12

### Herstellung von Ampullen

Ampullen, die die im folgenden genannten Bestandteile enthalten, lassen sich in bekannter Weise herstellen. Wirkstoff und Natriumchlorid werden in Wasser gelöst und unter Stickstoff in Glasampullen abgefüllt. Sie können zur Behandlung hypotoner Zustände in einer Dosis von 1 bis 2 Ampullen zweimal täglich verwendet werden.

| | |
|---|---|
| 2,3,3a,8-Tetrahydro-1H-pyrazolo/5,1-a/iso-indol-1-carboximidamid | 2,5 mg |
| Natriumchlorid | 18 mg |
| dest. Wasser ad | 2,0 ml |

## Beispiel 13

Nasentropfen, die die im folgenden genannten Bestandteile enthalten, lassen sich in bekannter Weise herstellen. Sie können in einer Dosis von zwei bis drei Tropfen pro Nasenloch zwei- bis viermal täglich verwendet werden.

| | |
|---|---|
| 2,3,3a,8-Tetrahydro-1H-pyrazolo/5,1-a/iso-indol-1-carboximidamid | 0,25 g |
| Methylcellulose | 1,0 g |
| dest. Wasser ad | 100 ml |

Patentansprüche

1. Substituierte Tetrahydro-1H-pyrazolo/5,1-a/isoindole der Formel I

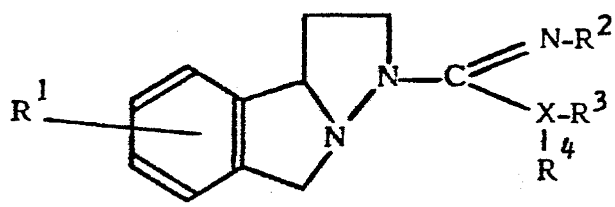

(I)

in der

$R^1$ ein Wasserstoff- oder Halogenatom oder eine Alkoxygruppe mit 1 bis 4 Kohlenstoffatomen,

$R^2$, $R^3$, $R^4$, die gleich oder verschieden sein können, ein Wasserstoffatom oder eine Alkylgruppe mit 1 bis 4 Kohlenstoffatomen oder

$R^2$ und $R^3$ zusammen die Äthylengruppe und

X ein Stickstoffatom oder eine Methingruppe bedeuten sowie deren Säureadditionssalze.

2. Verbindungen der Formel II

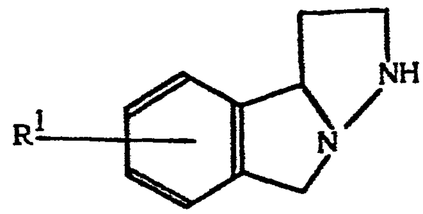

(II)

in der $R^1$ ein Wasserstoff- oder Halogenatom oder eine Alkoxygruppe mit 1 bis 4 Kohlenstoffatomen bedeutet und ihre Säureadditionssalze.

3. Verfahren zur Herstellung der Verbindungen gemäß Anspruch 1, dadurch gekennzeichnet, daß man Verbindungen der Formel II

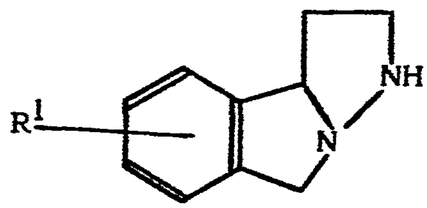

(II)

in der $R^1$ die oben angegebene Bedeutung hat, mit Verbindungen der allgemeinen Formel III umsetzt,

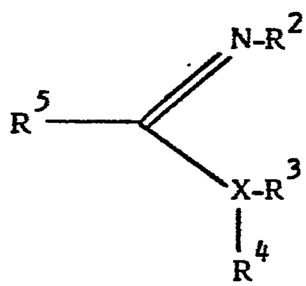

(III)

in der $R^2$, $R^3$ und $R^4$ die oben angegebene Bedeutung haben und in der $R^5$ eine nucleophil austauschbare Guppe wie die Alkylthio-oder Alkoxygruppe bedeutet.

4. Verfahren zur Herstellung der erfindungsgemäßen Verbindungen der Formel I gemäß Anspruch 1, in der $R^2$ Wasserstoff und X Stickstoff bedeutet, dadurch gekennzeichnet, daß man Verbindungen der Formel II, mit der angegebenen Bedeutung von $R^1$, mit Cyanamid oder mit substituierten Cyanamiden der Formel $NCNR^3R^4$, in der $R^3$ und $R^4$ die oben angegebene Bedeutung besitzen, umsetzt.

5. Verfahren zur Herstellung der Verbindungen der allgemeinen Formel II gemäß Anspruch 2, dadurch gekennzeichnet, daß man 2-Brommethylzimtsäure-alkylester der allgemeinen Formel IV

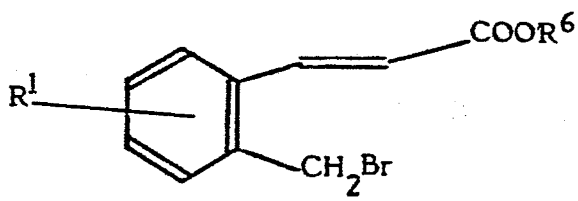

(IV)

in der $R^1$ die oben angegebene Bedeutung hat und $R^6$ eine Methyl- oder Ethylgruppe bedeutet, mit t-Butylcarbazat zu substituierten Isoindolinen der Formel V

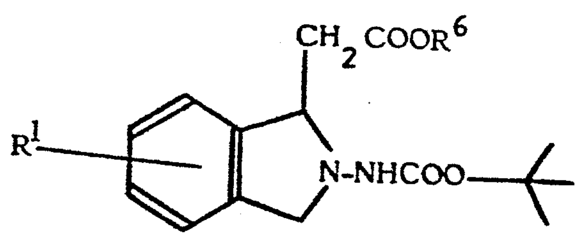

(V)

umsetzt und diese anschließend mit Säuren zu 2-Oxo-2,3,3a,8-tetrahydro-1H-pyrazolo/5,1-a/isoindolen der Formel (VI)

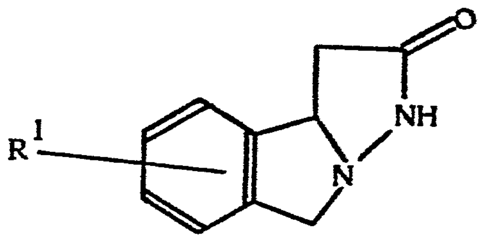

(VI)

cyclisiert und mit Reduktionsmitteln umsetzt.

6. Pharmazeutisches Präparat, dadurch gekennzeichnet, daß es eine oder mehrere der Verbindungen gemäß Anspruch 1 oder deren physiologisch verträgliche Salze und gegebenenfalls übliche Trägerstoffe und/oder Verdünnungsmittel enthält.

7. Verbindungen gemäß Anspruch 1 oder deren physiologisch verträglichen Salze zur Anwendung bei Hypotonie.

8. Verbindungen gemäß Anspruch 1 oder deren physiologisch verträglichen Salze zur Anwendung bei orthostatischer Dysregulation.

9. Verbindungen gemäß Anspruch 1 oder deren physiologisch verträglichen Salze zur Anwendung als Rhinologica.

Europäisches Patentamt

# EUROPÄISCHER RECHERCHENBERICHT

0126933
Nummer der Anmeldung

EP 84 10 4193

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | Chemical Abstracts Band 98, Nr. 3, 17. Januar 1983, Columbus, Ohio, USA; E. TOJA et al. "Synthesis and pregnancy terminating activity of 2-arylpyrazolo[5,1-a]isoindoles and isoquinolines", Seite 505, Spalte 1, Abstract Nr. 16614n & Eur. J. Med. Chem. - Chim. Ther., Band 17, Nr. 3, 1982, Seiten 223-227<br>----- | 2 | C 07 D 487/04<br>A 61 K 31/41 |
| | | | RECHERCHIERTE SACHGEBIETE (Int. Cl. 3) |
| | | | A 61 K 31/41<br>C 07 D 487/04 |

Der vorliegende Recherchenbericht wurde für alle Patentansprüche erstellt.

| Recherchenort | Abschlußdatum der Recherche | Prüfer |
|---|---|---|
| BERLIN | 25-07-1984 | KNAACK M |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82